# EUROPEAN PATENT SPECIFICATION

(11) **EP 1 455 802 B1**
(45) Date of publication and mention of the grant of the patent: **15.10.2008**
(21) Application number: 02787257.1
(22) Date of filing: 16.12.2002
(51) Int. Cl.: A61K 31/715, C08B 37/00

(54) **USES OF CHITOSAN OLIGOSACCHARIDES**
VERWENDUNGEN VON CHITOSANOLIGOSACCHARIDEN
UTILISATIONS D'OLIGOSACCHARIDES DE CHITOSANE

(30) Priority: 14.12.2001 CA 2364924
(43) Date of publication of application: 15.09.2004
(73) Proprietor: DNP CANADA INC., Granby QC J2H 2R6 (CA)
(72) Inventor: BOUCHER, Isabelle, Sherbrooke, Québec J1G 2K4 (CA); BRUNET, Serge, Sherbrooke, Québec J1G 2K4 (CA)
(74) Representative: Modiano, Micaela Nadia
(86) International application number: PCT/CA2002/001952
(87) International publication number: WO 2003/051376

(56) References cited:
- EP-A- 1 127 574
- WO-A-98/48627
- US-A- 4 532 134
- US-A- 5 730 876
- US-A- 5 891 861
- US-A1- 2001 001 788
- SKAUGRUD O: "CHITOSAN-NEW BIOPOLYMER FOR COSMETICS & DRUGS" DRUG AND COSMETIC INDUSTRY, XX, XX, vol. 148, 1 May 1991 (1991-05-01), page 24,26,30 XP000564874

## Description

### TECHNICAL FIELD

The present invention relates chitosan oligomer compositions for preventing a treating inflammation a hypersensitivity in a human or an animal.

### BACKGROUND OF THE INVENTION

An inflammation is a reaction of an organism following a traumatic stress, either chemical or microbial. Different signs or symptoms lead to identify an inflammation. These symptoms can vary from an uncomfortable cutaneous feeling, skin tension, itching to swelling, pain or redness and/or warmth sensation. Signs of inflammation may even be fever and/or general discomfort.

The symptoms of joint inflammation are generally associated with spondyloarthropathies (for example ankylosing spondylitis, psoriatic arthropathy, reactive arthritides and sacroiliitis) and rheumatoid arthritis. The joint inflammation is localised to different joints in these different conditions. In ankylosing spondylitis the inflammation is localised to the spine, the sacroiliac joints, and also often to the peripheral large joints (e.g. the knees, elbows and ankles). In sacroiliitis the inflammation is isolated to the sacroiliac joints but sometimes occurs in the peripheral joints as well. The other spondyloarthropathies have a similar clinical picture so far as which joints are inflamed. In rheumatoid arthritis a symmetrical joint inflammation occurs.

Rheumatoid arthritis and the spondyloarthropathies have in common a chronic inflammation of the synovial and extrasynovial structures such as the tendons and ligaments. The inflammatory reaction of the joints is dominated by certain inflammatory cells (for example neutrophils, activated lymphocytes and macrophages) which all contribute to the joint pain and the destruction of the joints.

The drugs which have in the past been used to treat joint inflammation are based on symptomatic anti-inflammatory treatments or disease modifying treatments. The dominating drugs for symptomatic treatments are non-steroidal anti-inflammatory drugs, orally active glucocorticosteroids with a mainly systemic effect or intraarticular injections of glucocorticosteroids. The disease modifying treatments include drugs which, by influencing the immune reactions of the body, reduce joint inflammation. Examples of disease modifying drugs include methotrexate, azathioprine, gold salts, cyclophosphamide and sulphasalazine. All of these treatments unfortunately cause severe side effects and are not particularly effective. For example glucocorticoid administration is generally directed against the local inflammation, i.e. it has been used to treat directly the inflammatory cells present in the joint inflammation. The routes used to administer such glucocorticoid treatment results in severe side effects on the body including effects on the skeleton and muscles.

Hypersensitivity is defined as a state of altered reactivity in which the body reacts with an exaggerated immune response to a substance (antigen). Hypersensitivity may be caused by exogenous or endogenous antigens.

Hypersensitivity reactions underlie a large number of diseases. Among these, allergic and autoimmune conditions are of great importance. A classification of hypersensitivity diseases is given in the textbook Clinical Medicine (Kumar, P. and Clark, M.: "Clinical Medicine", 3rd edition, p. 147-150, 1994, Bailliere Tindall, London).

Type I hypersensitivity reactions (IgE meditated allergic reactions) are caused by allergens (specific exogenous antigens), e.g. pollen, house dust, animal dandruff, and moulds. Allergic diseases in which type I reactions play a significant role include asthma, eczema (atopic dermatitis), urticaria, allergic rhinitis and anaphylaxis.

Type II hypersensitivity reactions are caused by cell surface or tissue bound antibodies (IgG and IgM) and play a significant role in the pathogenesis of myasthenia gravis, Good-pasture's syndrome and Addisonian pernicious anaemia.

Type III hypersensitivity reactions (immune complex) are caused by autoantigens or exogenous antigens, such as certain bacteria, fungi and parasite. Diseases in which type III hypersensitivity reactions play a significant role include lupus erythematosus, rheumatoid arthritis and glomerulonephritis.

Type IV hypersensitivity reactions (delayed) are caused by cell or tissue bound antigens. This type of hypersensitivity plays a significant role in a number of conditions, e.g. graft-versus-host disease, leprosy, contact dermatitis and reactions due to insect bites.

A number of drug classes are available for the treatment of hypersensitivity reactions. Some of these are applied systemically and some are applied topically.

The corticosteroids are among the most widely used drugs for the treatment of hyper-sensitivity diseases. Corticosteroids primarily exert their pharmacological action by non-selectively inhibiting the function and proliferation of different classes of immune cells resulting in suppression of hyper-sensitivity reactions. Unfortunately the corticosteroids are associated with a number of serious side effects, e.g. immuno-suppression, osteoporosis and skin atrophy (when applied topically)

Many individuals are affected by eczema or inflammation of the skin. Atopic dermatitis is the most severe and chronic form of eczema, although there are several other skin conditions that are eczemas including seborrheic dermatitis, irritant contact dermatitis, and allergic contact dermatitis. Skin inflammations may be triggered by any number of factors. For example, irritant contact (such as by solvents, chemicals, bacteria or detergents) may trigger eczema or acne. Eczema may also be triggered by allergens, for example by dermal exposure to plant species such as poison ivy, poison oak and poison sumac. Individuals with severe eczema such as atopic dermatitis are often prone to secondary skin infections such as by Staphylococcus bacteria or Herpes virus.

It is known in the art to treat inflammatory and pruritic manifestations of dermatitis syndromes topically with corticosteroids. The mechanism of anti-inflammatory actions of topical corticosteroids has not been completely elucidated. However, it is thought that corticosteroids act by inducing phospholipase A₂ inhibitory proteins called lipocortins. Lipocortins may control synthesis of potent mediators of inflammation such as prostaglandins and leukotrienes.

Heretofore, corticosteroid treatments for dermatitis have been topically applied in the form of creams gels, or lotions. These medicamentous vehicles tend to leave a greasy layer on the treated area which can be unpleasant to the recipient. Additionally, the task of preparing the appropriate suspension of active ingredients in a cream, lotion, or gel form can be laborious. Thus, the prior compositions of active medicaments, dispersed in their related delivery media, do not provide an ideal solution for treating dermal inflammation and irritation.

The pharmaceutical industry has searched for many years for substances that would treat and/or minimize inflammation symptoms and hypersensitivity. Even if a number of substances have been discovered, there is still needed to be provided with new products with anti-inflammatory and anti-hypersensitivity effects.

The present invention meets these needs as it will be evident to one skilled in the art when reading the description of the present invention.

### SUMMARY OF THE INVENTION

One object of the present invention is to provide chitosan oligomer compositions for preventing or treating inflammation or hypersensitivity in a human or an animal.

Chitosan is a linear 1,4-bound polysaccharide built up from β-D-glucosamine units. The chitosan is manufactured by N-deacetylation of chitin, a polymer forming the shell of insects and shellfish. Commercially, chitin is recovered from crab and shrimp shells which constitute waste products from the fishing industry. By controlling the alkaline treatment of chitins chitosans of varying degree of N-acetylation can be made. When treating chitin with concentrated alkali, usually sodium hydroxide, N-deacetylation thus takes place, i.e. acetamino groups are converted into amino groups to form chitosan.

The physical properties of chitosan affecting its usefulness depend on the degree of N-acetylation, the molecular weight and the homogeneity. Chitosan is bio-degradable, both by chitinase in the digestive system and by lysozyme and other enzymes in the body fluids.

The therapeutic compositions comprising said chitosan oligosaccharides may be used to prevent and/or treat topical inflammatory diseases, such as acne, psoriasis, or systemic diseases such as Crohn's disease, ulcerative colitis, articulation inflammation and arthrosis.

The cosmetic compositions comprising said chitosan oligosaccharides may be used for preparing lotions, gels or creams, for example sunscreens, antiallergic creams, hypoallergenic products, anti-wrinkle creams and anti-redness creams.

Said Chitosan oligosaccharides, allow the minimization or reduction of inflammations.

Said compositions are effective in treating the inflammation and irritation associated with skin disorders such as dermatitis with pruritic activity.

Said compositions are effective in treating inflammation and irritation associated with skin disorders such as dermatitis which incorporates a lower concentration of active ingredient, allowing longer-term treatment with reduced risk to the patient.

Additional objects, advantages and other novel features of the invention will be set forth in part in the description that follows and in part will become apparent to those skilled in the art upon examination of the following or may be learned with the practice of the invention. The objects and advantages of the invention may be realized and obtained by means of the instrumentalities and combinations particularly pointed out in the appended claims.

Many other objectives and advantages of the present invention will be discussed in the non restrictive description that follows.

### DETAILED DESCRIPTION OF THE PREFERRED EMBODIMENT

The present invention now will be described more fully hereinafter with reference to the accompanying drawings, in which preferred embodiments of the invention are shown. This invention, may, however, be embodied in many different forms and should not be construed as limited to the embodiments set forth herein; rather, these embodiments are provided so that this disclosure will be thorough and complete, and will fully convey the scope of the invention to those skilled in the art.

In accordance with the present invention, there is provided chitosan oligosaccharides with anti-inflammatory properties.

The expression "anti-inflammatory properties" means a process allowing chitosan oligosaccharides to minimize or reduce inflammatory responses.

A composition in accordance with the teachings of the present invention is provided for use in treating the inflammation and irritation of certain organs, such as for example, but not limited to, gut, intestine and skin. The composition is particularly effective in treating inflammation resulting from chronic, non-responsive allergic physiological reaction. Still for example, the physiological reaction that can be prevented or treated with the composition of the present invention and containing chitosan oligosaccharides, may include acquired or induced dermatitis, colitis, joint inflammation, skin or mucosal inflammation, sunburn, or Chrohn's disease

According to the present invention, chitosan oligosaccharides should be obtained from chitin with a degree of deacetylation varying between 75% and 100%.

Thus, the invention relates to deacetylated chitosan oligosaccharides with a low molecular weight less than 2000 Da.

According to the present invention, the chitosan oligosaccharides may be prepared and/or obtained by any appropriate process known by one skilled in the art. They may be obtained by the enzymatic method described in the United States patent number 5,482,843 and by the chemical method described by Horowitz, Roseman and Blumenthal (J, Amer. Chem. Soc., 1957, 79, 5046-5049).

The invention describes uses of chitosan oligosaccharides according to the present invention for the preparation of therapeutic and cosmetic compositions.

In accordance with the present invention, there is provided a composition comprising, in addition to chitosan oligomers, other polysaccharides, such as but not limited to, glycosaminoglycan (GAG) components (glucosamine, N-acetyl-glucosamine, galactosamine, N-acetylgalactosamine, glucuronate, iduronate, galactose), which can be extracted from biopolymers (such as chitin, chitosan, chondroitin, dermatan, keratan, heparin, hyaluronan). The oligosaccharide of the composition is easily absorbed through the gastrointestinal tract and uptaken through the circulatory system.

The oligomers of chitosan oligosaccharides provide an advantage as being the delivery vehicle for sustained release of GAG components. Another advantage is that the oligomers of monosaccharides ate metabolized more slowly compared to the monomers of monosaccharides. In this regard, the uptake of the raw components is reduced since the metabolic losses through mine, feces, breath and perspiration is less significant. There are multiple enzyme activities in the body fluids that can biodegrade the oligomers of monosaccharides and provide a sustained release format for monomers to the different connective tissues and articular cartilage. The molecular ranges and sizes of the molecule uptake are important for the bioavailability and delivery kinetics.

There is provided a method for preventing or treating all forms of inflammation by the delivery into organisms monomers of NAG and/or GS by the administration of short tandems containing between 2 to 50 units of NAG, monosaccharides, and/or GS in different proportions. It is intended with the invention to obtain targeted physiological effects by administering selected length of tandems of monosaccharides.

Preferable, the chitosan oligomers of the present invention comprise between 2 to 50 units of saccharide, and most preferentially between 2 to 25 units of saccharide.

These compositions can favorably help preventing or treating inflammatory diseases, such as those mentioned before. The preparation and administration methods of the compositions of the present invention are not described in detail because these are already known by one skilled in the art.

It will be recognized by those skilled in the art that the composition of the present invention containing partially or completely deacetylated chitosan oligomers or oligosaccharides can be administered as known by different ways, such as, for example, by oral, intradermal, intravenous, intranasal, subcutaneous, or topical administration.

It will also be recognized that the chitosan oligomers or oligosaccharides can be delivered under several forms, such as, but not limited to, pills, gels, creams, sprays or, in aqueous solution. For example, the composition containing chitosan oligosaccharides can be under the form of a cream, where they are mixed with emollients and other useful products in cosmetic and skin cares.

The present invention will be more readily understood by referring to the following examples which are given to illustrate the invention rather than to limit its scope.

### EXAMPLE I

### Introduction

The objectives of the present study are to evaluate the anti-inflammatory effects of two chitosan oligosaccharides (90D and 90E samples) according to the present invention, and, more particularly on prostaglandin E₂ (PGE₂) and IL1β release by NCTC human keratinocytes under a UVB irradiation.

Note: Two experiments were successively realized. Both experiments showed a very low induction stimulation of the sought markers following an infra-cytotoxic irradiation (250mj/cm2 UVB). In the second experiment, a stimulation trial by the pro-inflammatory agent "PMA" was realized.

### Materials and Methods

### Cells culture

Human keratinocytes were cultured in MEM/M199 (Gibco 31570021/2115130) culture medium 3:1, mixed with 1,87 mg/ml Sodium bicarbonate (Gibco 25080060), 2 mM L-glutamine (Gibco 25030024), 50 µl/ml Penicillin (Polyabo 60703), and 10% Fetal veal serum (v/v Gibco 10106151). Cell culture was performed at 37°C, in 5% CO₂ atmosphere.

### Evaluation of chitosan oligosaccharides 90D

- Deacetylation 90%
- Average molecular weight 700 Da
- Stock solution 25 mg/ml solution
- Dilutions in culture medium
- Tested final concentrations 250 µg/ml; 100 µg/ml; 10 µg/ml

### Evaluation of chitosan oligosaccharide 90E

- Deacetylation 90%
- Average molecular weight 1400 Da
- Stock solution 25 mg/ml solution
- Dilutions in culture medium
- Tested final concentrations 250 µg/ml; 100 µg/ml; 10 µg/ml
Reference 1 (PGE₂ induction): Indomethacin (Sigma 17378), tested at 1 µM (final)
Reference 2 (IL1β induction): Dexamethazone (Sigma D 1756), tested at 0.1 µM (final)

### Treatments and Assays

The treatments were realized in triplicate, with 6 plates, 96 identical wells after a 24-hour preculture.
Plate 1: No stimulation, PGE₂ assay (MTT viability)
Plate 2: UV stimulation, PGE₂ assay (MTT viability)
Plate 3: PMA stimulation, PGE₂ assay (MTT viability)
Plate 4: No stimulation, IL1β assay (MTT viability)
Plate 5: UV stimulation, IL1β assay (MTT viability)
Plate 6: PMA stimulation, IL1β assay (MTT viability)

The cells were cultured in presence of the products for a period of 24 hours. After the washout, the mediums were replaced by EBSS (buffered saline solution, GIBCO) and were irradiated or not by 250 mJ/cm² UVB (SOL500 lamp, H2 filter) Vilber-Lourmat radiometer, lamp calibrated just before exposition). Other plates were kept in the dark. Non-irradiated cultures were treated by a medium containing the products, with or without PMA (1 µg/ml phorbol, 12-myristate 13-acetate, Sigma P1585). After a culture of 24 hours, the cellular layer was observed and mediums collected and frozen. The cellular viability was evaluated by quantitation of the metabolic activity of cells (mitochondrial dehydrogenase) by hydrolysis measurement of MTT.

After thawing and centrifugation of the mediums, the content in PGE₂ was measured in ELISA with a R&D Systems kit (DE0100), according to the recommended protocol of the supplier. The IL1β content was measured in ELISA with a Immunotech kit (1042), according to the recommended protocol of the supplier.

### Data Treatment

The raw data were transformed and treated by the software PRISM® (Graph Pad Software). The intergroup comparisons were realized by analysis of variance (ANOVA), with the multiple comparison test DUNNETT.

### Results and Conclusion

### Viability

The viability measures (%, MTT test) are reported in the tables. UV irradiation did not significantly reduce the cellular viability. The 250 mJ/cm2 UVB irradiation used was determined as only being infra-cytotoxic in experimental conditions (viability reduction of less than 10%).

Also, cells treatment with PMA did not significantly modify the viability. In any conditions, the 90D and 90E products did not significantly modify the cellular viability.

### PGE₂ evaluation

Table 1 illustrates the effects of different treatments on the PGE₂ release in the non-treated or treated mediums by the UV or PMA.

**Table 1**

| **Effects of different treatments on the PGE₂ release in the non-treated or treated mediums by the UV or PMA; standard deviation (sd)** | | | | | |
|---|---|---|---|---|---|
| No stimulation | | | | | |
| *Treatment* | **PGE2 (pg/ml)** | **sd** | **N** | **%** | **Viability %** |
| Control | **11.0** | 3 | 3 | **100** | 100 |
| Indomethacin | **0.9** | 3 | 3 | **8** | 120 |
| 90D 250 µg/ml | **8.6** | 2 | 3 | **78** | 109 |
| 90D 100 µg/ml | **6.9** | 3 | 3 | **62** | 104 |
| 90D 10 µg/ml | **14.0** | 8 | 3 | **127** | 121 |
| 90E 250 µg/ml | **9.7** | 4 | 3 | **88** | 110 |
| 90E 100 µg/ml | **10.1** | 1 | 3 | **92** | 99 |
| 90E 10 µg/ml | **4.7** | 6 | 3 | **43** | 95 |

| UVB Stimulation | | | | | |
|---|---|---|---|---|---|
| *Treatment* | **PGE2 (pg/ml)** | **sd** | **N** | **%** | **Viability %** |
| Control | **33.8** | 15 | 3 | **100** | 100 |
| Indomethacin | **7.3** | 7 | 3 | **22** | 117 |
| 90D 250 µg/ml | **9.5** | 9 | 3 | **28** | 101 |
| 90D 100 µg/ml | **17.7** | 4 | 3 | **52** | 98 |
| 90D 10 µg/ml | **24.4** | 7 | 3 | **72** | 105 |
| 90E 250 µg/ml | **17.2** | 6 | 3 | **51** | 108 |
| 90E 100 µg/ml | **17.8** | 2 | 3 | **53** | 113 |
| 90E 10 µg/ml | **20.2** | 3 | 3 | **60** | 103 |

| PMA Stimulation | | | | | |
|---|---|---|---|---|---|
| *Treatment* | **PGE2 (pg/ml)** | **sd** | **N** | **%** | **Viability %** |
| Control | **4852** | 12 | 3 | **100** | 100 |
| Indomethacin | **10** | 16 | 3 | **0** | 111 |
| 90D 250 µg/ml | **4193** | 157 | 3 | **86** | 103 |
| 90D 100 µg/ml | **4641** | 23 | 3 | **96** | 100 |
| 90D 10 µg/ml | **4722** | 12 | 3 | **97** | 105 |
| 90E 250 µg/ml | **4469** | 23 | 3 | **92** | 101 |
| 90E 100 µg/ml | **4660** | 17 | 3 | **96** | 100 |
| 90E 10 µg/ml | **4679** | 24 | 3 | **96** | 98 |

### Non-stimulated controls

A low PGE₂ quantity (10 pg/ml) was present in control medium at the end of the experiment. This low concentration leads to the fluctuation of measures and to non-significant statistic analysis.

The indomethacin treatment has apparently totally blocked the production/release of this basal level.

### UV Stimulation

The UV stimulated the PGE₂ production with a factor 3; the values remaining low and standard deviations high.

The indomethacin significantly reduced the PGE₂ release.

The different tested products apparently had a global trend to reduce PGE₂ release.

### PMA Stimulation

PMA treatment, as anticipated, highly stimulated PGE₂ release. Thus, the results are highly reproducible and internal variability very low.

The indomethacin cyclooxygenase inhibitor totally blocked the PGE₂ production/release.

The 90D and 90E products have slightly reduced the PGE₂ release induced by PMA (86-97% of control PMA). Because of the high PGE₂ quantity and of the low variability of results, this leads to confirm the observed effect during UV stimulation.

**Table 2**

| **Effects of the different treatments on ILIβ release in treated or non-treated mediums by UV or PMA; sd** | | | | | | |
|---|---|---|---|---|---|---|
| No stimulation | | | | | | |
| *Treatment* | **IL1β (pg/ml)** | **sd** | **N** | **%** | **Viability %** | |
| Control | **nd** | --- | --- | **100** | 100 | |
| dexamethasone | **nd** | --- | --- | **8** | 100 | |
| 90D 250 µg/ml | **nd** | --- | --- | **78** | 110 | |
| 90D 100 µg/ml | **nd** | --- | --- | **62** | 107 | |
| 90D 10 µg/ml | **nd** | --- | --- | **127** | 118 | |
| 90E 250 µg/ml | **nd** | --- | --- | **88** | 100 | |
| 90E 100 µg/ml | **nd** | --- | --- | **92** | 116 | |
| 90E 10 µg/ml | **nd** | --- | --- | **43** | 112 | |

| UVB Stimulation | | | | | | |
|---|---|---|---|---|---|---|
| *Treatment* | **IL1β (pg/ml)** | **sd** | **N** | **%** | **P** | **Viability %** |
| Control | **nd** | --- | --- | --- | --- | --- |
| dexamethasone | **nd** | --- | --- | --- | --- | 96 |
| 90D 250 µg/ml | **nd** | --- | --- | --- | --- | 118 |
| 90D 100 µg/ml | **nd** | --- | --- | --- | --- | 101 |
| 90D 10 µg/ml | **nd** | --- | --- | --- | --- | 120 |
| 90E 250 µg/ml | **nd** | --- | --- | --- | --- | 118 |
| 90E 100 µg/ml | **nd** | --- | --- | --- | --- | 123 |
| 90E 10 µg/ml | **nd** | --- | --- | --- | --- | 112 |

| PMA Stimulation | | | | | | |
|---|---|---|---|---|---|---|
| *Treatment* | **IL1β (pg/ml)** | **sd** | **N** | **%** | **P** | **Viability %** |
| Control | **3,2** | 1 | 3 | 100 | --- | 100 |
| dexamethasone | **0,2** | 0 | 3 | 5 | <0.01 | 92 |
| 90D 250 µg/ml | **1,7** | 1 | 3 | 53 | <0.01 | 101 |
| 90D 100 µg/ml | **1,3** | 0 | 3 | 41 | <0.01 | 103 |
| 90D 10 µg/ml | **2,5** | 1 | 3 | 76 | >0.01 | 102 |
| 90E 250 µg/ml | **2,7** | 0 | 3 | 85 | >0.05 | 98 |
| 90E 100 µg/ml | **1,6** | 0 | 3 | 51 | <0.05 | 98 |
| 90E 10 µg/ml | **2,6** | 1 | 3 | 75 | >0.05 | 94 |

Table 2 illustrates the effects of the different treatment on IL1β release in treated or non-treated mediums by UV or PMA.

### Non stimulated controls

The IL1β was not detectable by the method used, in non-stimulated supernatant mediums.

### UV Stimulation

The UV did not lead to a significant release (measurable) of IL1β. The UV dose used was maximal in terms of limit of effect on cellular viability. These results confirm the results of a first experiment realized with lower UV doses. UV cannot apparently release NCTC keratinocytes of IL1β, in these experimental conditions. Following this unsuccessful attempt, a trial of secretion stimulation of IL1β by the PMA was initiated.

### PMA Stimulation

PMA treatment stimulated the IL1β release, since this cytokine was detectable in supernatants of treated cells. However, the IL1β concentration was very low (3 pg/ml) and the stimulation was then very different to the one observed for PGE₂.

The dexamethasone reference significantly inhibited the production/release of IL1β.

The 90D and 90E products reduced the IL1β release induced by the PMA without net dose-effect.

## Claims

1. A chitosan oligomer composition for preventing or treating inflammation or hypersensitivity in a human or an animal, said composition being for oral, intradermal, intravenous, intranasal, subcutaneous or topical administration and said chitosan oligomer being obtained from chitin at least 75% deacetylated and having a molecular weight less than 2000 Da.

2. The chitosan oligomer composition of claim 1, wherein said composition is a therapeutic, a cosmetic or a nutraceutic composition.

3. The chitosan oligomer composition of claim 1 or 2, **characterized in that** said inflammation is caused by trauma, sunburn, heat eczema, contact allergy, psoriasis, eypsipelas, acne, nail inflammation, cuts, bums, insect bites, insect stings, pruritus, autoimmune reaction, rheumatoid reaction or arthritic reaction.

4. The chitosan oligomer composition of claim 1 or 2, **characterized in that** said inflammation is present in joint inflammation, arthrosis, skin or mucosal inflammation, ulcerative colitis, or Crohn's disease.

5. The chitosan oligomer composition of claim 1 or 2, **characterized in that** said hypersensitivity is caused by allergic reactions caused by allergens selected from the group consisting of pollen, house dust, animal dandruff, and moulds.

6. The chitosan oligomer composition of claim 1 or 2, **characterized in that** said hypersensitivity is present in asthma, eczema, atopic dermatitis, urticaria, allergic rhinitis and anaphylaxis.

7. The chitosan oligomer composition of claim 1 or 2, **characterized in that** said hypersensitivity is caused by cell surface or tissue bound antibodies, autoantigens, exogenous antigens, bacteria, fungi, or parasites.

8. The chitosan oligomer composition of claim 1 or 2, **characterized in that** said hypersensitivity is present in myasthenia gravis, Good-pasture's syndrome, Addisonian pernicious anaemia, lupus erythematosus, rheumatoid arthritis, glomerulonepthritis, delayed hypersensitivity reactions, graft related diseases or leprosy.

9. The chitosan oligomer composition of claim 1, wherein said chitosan oligomer has an average molecular weight of about 700 kDa and is obtained from chitin 90% deacetylated.

10. The chitosan oligomer composition of claim 1, wherein said chitosan oligomer has an average molecular weight of about 1400 kDa and is obtained from chitin 90% deacetylated.

11. The chitosan oligomer composition of claim 1, wherein said chitosan is completely deacetylated.

## Patentansprüche

1. Chitosanoligomer-Zusammensetzung zur Vorbeugung oder Behandlung von Entzündung oder Überempfindlichkeit bei einem Menschen oder einem Tier, wobei die Zusammensetzung zur oralen, intradermalen, intravenösen, intranasalen, subkutanen oder lokalen Verabreichung ist und das Chitosanoligomer aus Chitin gewonnen wird, das zu mindestens 75% entacetyliert ist und ein Molekulargewicht von weniger als 2000 Da hat.

2. Chitosanoligomer-Zusammensetzung gemäß Anspruch 1, worin die Zusammensetzung eine therapeutische, eine kosmetische oder eine nutrazeutische Zusammensetzung ist.

3. Chitosanoligomer-Zusammensetzung gemäß Anspruch 1 oder 2, **dadurch gekennzeichnet, dass** die Entzündung verursacht wird durch Trauma, Sonnenbrand, Hitzeekzem, Kontaktallergie, Schuppenflechte, Erysipel(ββ), Akne, Nagelentzündung, Schnitte, Verbrennungen, Insektenbisse, Insektenstiche, Pruritus, Autoimmunreaktion, rheumatoide Reaktion oder arthritische Reaktion.

4. Chitosanoligomer-Zusammensetzung gemäß Anspruch 1 oder 2, **dadurch gekennzeichnet, dass** die Entzündung vorhanden ist in Gelenkentzündung, Arthrose, Haut- oder Schleimhautentzündung, Colitis ulcerosa oder Morbus Crohn.

5. Chitosanoligomer-Zusammensetzung gemäß Anspruch 1 oder 2, **dadurch gekennzeichnet, dass** die Überempfindlichkeit verursacht wird durch allergische Reaktionen, verursacht durch Allergene gewählt aus der Gruppe bestehend aus Pollen, Hausstaub, Tierschuppen und Schimmel.

6. Chitosanoligomer-Zusammensetzung gemäß Anspruch 1 oder 2, **dadurch gekennzeichnet, dass** die Überempfindlichkeit vorhanden ist in Asthma, Ekzem, Atopik-Dermatitis, Nesselsucht, allergischer Rhinitis und Anaphylaxie.

7. Chitosanoligomer-Zusammensetzung gemäß Anspruch 1 oder 2, **dadurch gekennzeichnet, dass** die Überempfindlichkeit verursacht wird durch an die Zelloberfläche oder ans Gewebe gebundene Antikörper, Autoantigene, exogene Antigene, Bakterien, Pilze oder Parasiten.

8. Chitosanoligomer-Zusammensetzung gemäß Anspruch 1 oder 2, **dadurch gekennzeichnet, dass** die Überempfindlichkeit vorhanden ist in Myasthenia gravis, Goodpasture-Syndrom, perniziöser Addison-Anämie, Lupus erythematodes, Rheumatoidarthritis, Glomerulonephritis, verzögerten Überempfindlichkeitsreaktionen, transplantatbezogenen Krankheiten oder Lepra.

9. Chitosanoligomer-Zusammensetzung gemäß Anspruch 1, worin das. Chitosanoligomer ein durchschnittliches Molekulargewicht von ungefähr 700 kDa hat und aus zu 90% entacetyliertem Chitin gewonnen wird.

10. Chitosanoligomer-Zusammensetzung gemäß Anspruch 1, worin das Chitosanoligomer ein durchschnittliches Molekulargewicht von ungefähr 1400 kDa hat und aus zu 90% entacetyliertem Chitin gewonnen wird.

11. Chitosanoligomer-Zusammensetzung gemäß Anspruch 1, worin das Chitosan vollständig entacetyliert ist.

## Revendications

1. Composition oligomère de chitosan pour prévenir ou traiter l'inflammation ou l'hypersensibilité chez un humain ou un animal, ladite composition étant destinée à une administration orale, intradermique, intraveineuse, intranasale, sous-cutanée ou topique et ledit oligomère de chitosan étant obtenu à partir de chitine désacétylée à au moins 75 % et ayant un poids moléculaire inférieur à 2 000 Da.

2. Composition oligomère de chitosan selon la revendication 1, ladite composition étant une composition thérapeutique, cosmétique ou nutraceutique.

3. Composition oligomère de chitosan selon la revendication 1 ou 2, **caractérisée en ce que** ladite inflammation est causée par un traumatisme, un coup de soleil, la chaleur, l'eczéma, une allergie de contact, le psoriasis, l'érysipèle, l'acné, une inflammation d'un ongle, des coupures, des brûlures, des piqûres d'insecte, des dards d'insecte, un prurit, une réaction auto-immune, une réaction rhumatoïde ou une réaction arthritique.

4. Composition oligomère de chitosan selon la revendication 1 ou 2, **caractérisée en ce que** ladite inflammation est présente dans une inflammation articulaire, une arthrose, une inflammation cutanée ou des muqueuses, la rectocolite hémorragique ou la maladie de Crohn.

5. Composition oligomère de chitosan selon la revendication 1 ou 2, **caractérisée en ce que** ladite hypersensibilité est causée par des réactions allergiques causées par des allergènes choisis dans le groupe consistant en les pollens, la poussière domestique, les phanères d'animaux et les moisissures.

6. Composition oligomère de chitosan selon la revendication 1 ou 2, **caractérisée en ce que** ladite hypersensibilité est présente dans l'asthme, l'eczéma, la dermatite atopique, l'urticaire, la rhinite allergique et l'anaphylaxie.

7. Composition oligomère de chitosan selon la revendication 1 ou 2, **caractérisée en ce que** ladite hypersensibilité est causée par des anticorps à la surface de cellules ou liés à un tissu, des autoantigènes, des antigènes exogènes, des bactéries, des champignons ou des parasites.

8. Composition oligomère de chitosan selon la revendication 1 ou 2, **caractérisée en ce que** ladite hypersensibilité est présente dans la myasthénie grave, le syndrome de Goodpasture, l'anémie pernicieuse d'Addison, le lupus érythémateux, la polyarthrite rhumatoïde, la glomérulonéphrite, les réactions d'hypersensibilité retardée, les maladies liées aux greffes ou la lèpre.

9. Composition oligomère de chitosan selon la revendication 1, dans laquelle ledit oligomère de chitosan a un poids moléculaire moyen d'environ 700 kDa et est obtenu à partir de chitine désacétylée à 90 %.

10. Composition oligomère de chitosan selon la revendication 1, dans laquelle ledit oligomère de chitosan a un poids moléculaire moyen d'environ 1 400 kDa et est obtenu à partir de chitine désacétylée à 90 %.

11. Composition oligomère de chitosan selon la revendication 1, dans laquelle ledit chitosan est complètement désacétylé.
